(19)

Europäisches
Patentamt
European
Patent Office
Office européen
des brevets

(11) **EP 3 505 072 A1**

(12) **EUROPEAN PATENT APPLICATION**

(43) Date of publication:
**03.07.2019 Bulletin 2019/27**

(51) Int Cl.:
***A61B 8/08*** (2006.01)

(21) Application number: **17210910.0**

(22) Date of filing: **28.12.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventor: **JIN, Sheng
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **IMPROVING SOUND PLAYING QUALITY FOR A FETAL MONITOR**

(57)     A Doppler ultrasound device (10) with an ultrasound transducer (12) generates a digitized demodulated Doppler ultrasound signal (40). The Doppler ultrasound device generates a higher pitched digitized demodulated Doppler ultrasound signal (50) by signal segment downsampling operations (52) producing downsampled signal segments and duplication operations (54) that duplicate the downsampled signal segments. A loudspeaker (20) outputs the higher pitched digitized demodulated Doppler ultrasound signal as an audible Doppler ultrasound signal. Each downsampling operation may be by a factor of $N$ where $N$ is a positive integer greater than or equal to two, and duplication operations generate duplicates of the downsampled signal segment to fill the original time interval. In fetal monitoring applications, digital signal processing (DSP) (42) is performed on the digitized demodulated Doppler ultrasound signal to detect at least one of a fetal heart rate and a fetal movement count that is presented on a display (18).

Fig. 1

EP 3 505 072 A1

## Description

### FIELD

The following relates generally to the medical ultrasound arts, fetal monitor arts, and related arts.

### BACKGROUND

An ultrasound-based fetal monitor employs Doppler ultrasound to monitor a fetus in the womb. The device includes an ultrasound transducer array that sonicates the womb at ultrasonic frequency typically in the megahertz range. The reflected Doppler ultrasound signal is detected by the same or a different transducer array, pre-amplified, demodulated to an intermediate frequency (IF) in the audio frequency range, and the demodulated Doppler ultrasound signal is digitized by sample-and-hold analog-to-digital (A/D) circuitry to generate a digitized demodulated Doppler ultrasound signal. The signal processing chain may include variant and/or other operations such as quadrature IQ demodulation, harmonic and/or sideband filtering, and/or so forth, and the processing may be variously partitioned between the analog or digital domains. Digital signal processing (DSP) is applied to the digitized demodulated Doppler ultrasound signal to extract clinically useful information. For example, envelope filtering may be applied to identify signal features of a few hertz or lower that are attributable to the fetal heartbeat and/or fetal movements, additional analyses may be employed to suppress potentially confounding signals due to the maternal heartbeat, hiccups, or so forth.

In addition to quantitative DSP performed on the digitized demodulated Doppler ultrasound signal, it is known to feed the demodulated Doppler ultrasound signal to an audio amplifier/loudspeaker chain. Because the demodulated Doppler ultrasound signal is in the audio frequency range, the output of the loudspeaker is an audible acoustic signal. The listener hears sounds in this audible signal that correlate in time with the fetal heartbeat, fetal movements, or other features in the demodulated Doppler ultrasound signal of the types that may be quantitatively analyzed by the DSP. The audio output is not a quantitative clinical result, but does have numerous uses. The sonographer, ultrasound technician, or other medical professional operating the fetal monitor commonly utilizes the audio output to position the ultrasound transducer optimally to detect the fetal heartbeat. An experienced medical professional may also be able to derive diagnostic information from the audio signal (which may be confirmed by quantitative DSP analyses). Furthermore, the expectant parent(s) are often aware ahead of time that they can expect to "hear" the fetal heartbeat during a scheduled ultrasound examination, and this experience is comforting and encouraging for the expectant parent(s).

While fetal monitoring is described herein as an illustrative clinical application, other medical Doppler ultrasound devices and procedures commonly utilize the described audio output. Some other examples include echocardiography and vascular ultrasound procedures. The audio output is again useful to the medical professional in placement of the ultrasound transducer and aurally confirming operation of the ultrasound device. Furthermore, if the patient is awake and not sedated during such procedures, the patient can likewise hear the audio output, and the medical professional can therefore use this as a teaching tool to explain about diagnostic information being obtained by the ultrasound procedure.

The following discloses certain improvements.

### SUMMARY

In some embodiments disclosed herein, a Doppler ultrasound method for improving signal quality of a signal to be played comprises: detecting a cycle of a digitized demodulated Doppler ultrasound signal; downsampling the detected cycle to generate a downsampled cycle; duplicating the downsampled cycle to fill a time interval of the detected cycle; and repeating the detecting, downsampling, and duplicating for a plurality of cycles to generate a higher pitched digitized demodulated Doppler ultrasound signal. In some embodiments, the downsampling is by a factor $of\ N$ where $N$ is a positive integer with a value of $N = 2$ or higher, and the duplicating comprises generating duplications of the downsampled cycle to fill the time interval of the detected cycle. In some embodiments suitable for fetal heart monitoring, digital signal processing (DSP) is also performed on the digitized demodulated Doppler ultrasound signal to determine quantitative fetal information including at least one of a fetal heart rate and a fetal movement count, and the quantitative fetal information is presented on a display.

In some embodiments the digitized demodulated Doppler ultrasound signal is generated at an intermediate frequency by demodulating and digitizing a Doppler ultrasound signal. In such embodiments, upon setting the intermediate frequency of the demodulating, the factor $of\ N$ for the downsampling is selected on the basis of the intermediate frequency to set a pitch of the higher pitched digitized demodulated Doppler ultrasound signal.

In some embodiments disclosed herein, a medical device includes an ultrasound transducer and a Doppler ultrasound device operatively connected with the ultrasound transducer to generate a digitized demodulated Doppler ultrasound signal from a Doppler ultrasound signal acquired by the ultrasound transducer. The Doppler ultrasound device includes at least one electronic processor programmed to generate a higher pitched digitized demodulated Doppler ultrasound signal from the digitized demodulated Doppler ultrasound signal by signal segment downsampling operations producing downsampled signal segments and duplication operations that duplicate the downsampled signal segments. A loudspeaker is connected to output the higher pitched digitized demodulated Doppler ultrasound signal as an audible Doppler ultrasound signal. In some embodiments, each downsampling operation downsamples a signal segment occupying a time interval by a factor *of N* where *N* is a positive integer greater than or equal to two, and duplication operations generate duplicates of the downsampled signal segment to fill the time interval of the signal segment. The medical device may further include a display, wherein the at least one electronic processor is further programmed to perform digital signal processing (DSP) on the digitized demodulated Doppler ultrasound signal to determine quantitative fetal information including at least one of a fetal heart rate and a fetal movement count and to operate the display to present the quantitative fetal information.

One advantage resides in providing a medical ultrasound device with audio output having improved sound quality.

Another advantage resides in providing a medical ultrasound device with audio output having improved sound quality while retaining the informational sound content of the audio output.

Another advantage resides in providing a medical ultrasound device with audio output having adjustable audible pitch.

Another advantage resides in providing an ultrasound-based fetal monitor with one or more of the foregoing advantages. Another advantage resides in providing an echocardiography device with one or more of the foregoing advantages. Another advantage resides in providing a vascular ultrasound device with one or more of the foregoing advantages.

A given embodiment may provide none, one, two, more, or all of the foregoing advantages, and/or may provide other advantages as will become apparent to one of ordinary skill in the art upon reading and understanding the present disclosure.

**BRIEF DESCRIPTION OF THE DRAWINGS**

The invention may take form in various components and arrangements of components, and in various steps and arrangements of steps. The drawings are only for purposes of illustrating the preferred embodiments and are not to be construed as limiting the invention.

Fig. 1 diagrammatically illustrates an ultrasound-based fetal monitor, with electronic processor diagrammatically represented.
Fig. 2 diagrammatically illustrates a digitized demodulated Doppler ultrasound signal (with +2000 offset) and the digitized demodulated Doppler ultrasound signal after processing to improve audio quality as disclosed herein.
Fig. 3 plots the spectral density of the digitized demodulated Doppler ultrasound signal of Fig. 2 (without the +2000 offset).
Fig. 4 plots the spectral density of the digitized demodulated Doppler ultrasound signal after processing to improve audio quality of Fig. 2.
Fig. 5 diagrammatically illustrates one detected cycle of the intermediate frequency (IF) waveform of a digitized demodulated Doppler ultrasound signal extending from one negative-to-positive zero crossing to the next negative-to-positive zero crossing.
Fig. 6 diagrammatically illustrates the detected cycle of Fig. 5 after downsampling by a factor *of N* = 3.
Fig. 7 diagrammatically illustrated the downsampled cycle of Fig. 6 after (*N* - 1) duplications.
Fig. 8 diagrammatically illustrates one detected cycle of the IF waveform of a digitized demodulated Doppler ultrasound signal extending from one maximum to the next maximum.
Fig. 9 diagrammatically illustrates the detected cycle of Fig. 8 after downsampling by a factor *of N* = 3.
Fig. 10 diagrammatically illustrated the downsampled cycle of Fig. 9 after (*N* - 1) duplications.
Fig. 11 diagrammatically illustrates one detected half-cycle of the IF waveform of a digitized demodulated Doppler ultrasound signal extending from a negative-to-positive zero crossing to a positive-to-negative zero crossing.
Fig. 12 diagrammatically illustrates the detected half-cycle of Fig. 11 after downsampling by a factor of *N* = 4.
Fig. 13 diagrammatically illustrated the downsampled half-cycle of Fig. 12 after (*N* - 1) duplications.
Fig. 14 diagrammatically illustrated the downsampled half-cycle of Fig. 12 after (*N* - 1) duplications with the first and third duplications also inverted by multiplication by a factor of -1.

**DETAILED DESCRIPTION**

Sound quality of the audio output of an ultrasound-based fetal monitor or other medical ultrasound device is of significance to the medical professional operating the medical ultrasound device, and may also be of significance to the expectant parent or other patient undergoing an ultrasound procedure. The sounds contained in the audio output (e.g. sounds correlating with fetal heartbeats, sounds correlating with fetal movements, sounds correlating with cardiac valve cycling, sounds correlating with vascular blood flow, et cetera) should maintain time synchronization with the underlying physiological sources, and these sounds should be sufficiently distinct and loud to be readily perceived by the listener, even in a possibly noisy hospital or clinical environment. For the patient, it may also be beneficial for the sounds to be aesthetically pleasing.

The pitch of the audible output corresponds to the intermediate frequency (IF) of the demodulated Doppler ultrasound signal, and is typically in the audio frequency range, i.e. about 20 Hz to 20 kHz for a typical adult with normal hearing. However, the IF is usually at the low end of this range, e.g. around 120 Hz in some illustrative examples herein. This is perceived as a very low audible pitch, that is, a deep or low-pitched droning sound. This low-pitched sound is perceived as unpleasant by many people. Additionally, persons with significant hearing loss at the lower end of the audio frequency range may have difficulty accurately hearing this low-pitched sound, or if heard may have difficult identifying subtle features of the sound.

Various approaches could in principle be used to improve the audio quality by shifting the pitch of the signal higher into the audio range. A simple expedient is to take the absolute value of the demodulated Doppler ultrasound signal, which provides roughly a doubling in frequency. However, this approach cannot be tailored to other frequency shifts besides doubling, and moreover a sharp inflection (very large second derivative) is generated at the zero point as the slope is abruptly reversed. On the other hand, complex pitch shifting algorithms operating in the frequency domain add substantial complexity and may be difficult to implement in real time. Yet another possible solution is to adjust the intermediate frequency (IF) of the demodulation to a (usually higher) frequency that sounds more pleasing when output by a loudspeaker. However, this can be an unsatisfactory solution since increasing the audio pitch would involve increasing the IF which in turn would require higher speed downstream analog-to-digital converters and a higher sampling rate, thus increasing hardware cost/complexity.

Approaches disclosed herein generate a higher pitched digitized demodulated Doppler ultrasound signal from the digitized demodulated Doppler ultrasound signal by signal segment downsampling operations producing downsampled signal segments and duplication operations that duplicate the downsampled signal segments. The higher pitched digitized demodulated Doppler ultrasound signal is then sent to the audio amplifier/loudspeaker chain. In one illustrative approach, the higher pitched digitized demodulated Doppler ultrasound signal is generated by detecting a cycle (or, in some alternative embodiments, a cycle fraction) of an IF waveform of the digitized demodulated Doppler ultrasound signal, downsampling the detected cycle to generate a downsampled cycle, and duplicating the downsampled cycle to fill a time interval of the detected cycle of the IF waveform. This process of detecting, downsampling, and duplicating is repeated for a plurality (or succession) of cycles of the IF waveform to generate the higher pitched digitized demodulated Doppler ultrasound signal.

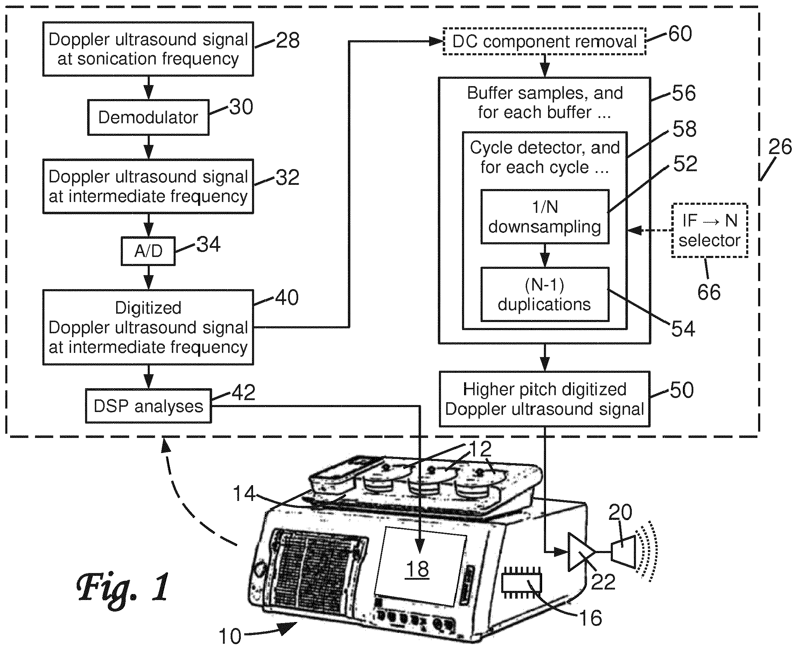

With reference to Fig. 1, an illustrative Doppler ultrasound device **10** includes an ultrasonic transducer **12**. More particularly, illustrative Fig. 1 shows three ultrasound transducers **12** disposed on a tray or receptacle **14** so as to be conveniently available for use with the Doppler ultrasound device **10** in a maternity ward or other medical setting likely to have multiple patients. The ultrasonic transducer **12** is configured to acquire a Doppler ultrasound signal. For example, the ultrasound transducer **12** can be secured or otherwise attached to an abdominal area of an expectant parent (not shown) carrying a fetus (not shown) such that the ultrasonic transducer **12** overlies a portion of the fetus. Some non-limiting illustrative examples of suitable Doppler ultrasound devices include the Philips Avalon™ series fetal monitors with associated wired or wireless ultrasound transducer accessories.

The Doppler ultrasound device **10** further includes typical components, such as at least one electronic processor **16** (internal component diagrammatically indicated in Fig. 1), various user-operable controls for performing setup and control of a monitoring session, and a display **18**. In some embodiments, the display can be a separate component from the Doppler ultrasound device **10** with a wired or wireless operational connection, and/or multiple displays may be provided, e.g. one for user interfacing and another to display acquired ultrasound images. The display **18** is operated by the electronic processor **16** to display ultrasound images or data, and/or to display clinically useful information extracted from ultrasound data by digital signal processing (DSP) performed by the at least one electronic processor **16**. For example, if a Doppler ultrasound signal is acquired of a fetus, then envelope filtering may be applied to identify signal features of a few hertz or lower that are attributable to the fetal heartbeat and/or fetal movements, additional analyses may be employed to suppress potentially confounding signals due to the maternal heartbeat, hiccups, or so forth. Fetal movement information may be displayed in various ways, such as a count of fetal movements over a defined time interval, which may be rolling in time (e.g., displaying the number of fetal movements detected in the last three minutes). The at least one electronic processor **16** may, for example, comprise a microprocessor, a microcontroller, a field programmable gate array (FPGA), or a combination thereof, along with ancillary electronics such as random access memory (RAM), ancillary discrete components (resistors, capacitors, and/or inductors), and/or so forth. The illustrative Doppler ultrasound device **10** further includes a loudspeaker **20** driven by an audio amplifier **22** (which in some embodiments may be built into the loudspeaker **20**). It is again to be appreciated that, as with the at least one electronic processor **16**, the loudspeaker **20** and audio amplifier **22** are typically internal components of the Doppler ultrasound device **10** which are diagrammatically shown in Fig. 1 but are not usually visible, or in the case of the loudspeaker **20** may be visible only via openings (i.e. "sound holes") in the housing of the Doppler ultrasound device **10** provided to enhance sound output.

With continuing reference to Fig. 1, an upper inset **26** illustrates internal analog processing components and DSP implemented by radio frequency (RF) circuitry and/or the at least one electronic processor **16**. An analog Doppler ultrasound signal **28** is acquired by the ultrasound transducer **12** at a sonication frequency that is ultrasonic, i.e., above the upper threshold of hearing, i.e. ~20 kHz, and more typically in the megahertz range. More particularly, an ultrasound transducer array of the transducer **12** sonicates the womb (for fetal monitoring) at an ultrasonic frequency under control of the Doppler ultrasound device **10,** and the reflected Doppler ultrasound waves are detected by the same or a different transducer array of the ultrasound transducer **12.** The detected signal is usually pre-amplified by an on-board amplifier of the ultrasound transducer **12,** and transmitted to the Doppler ultrasound device **10** as the Doppler ultrasound signal **28.** This Doppler ultrasound signal **28** is demodulated by a demodulator **30** to generate a Doppler ultrasound signal **32** at an intermediate frequency (IF), which is usually in the audio frequency range. The demodulator **30** may comprise RF circuitry or components such as an analog heterodyne mixer, e.g. implemented as an RF component integrated circuit (IC), followed by analog harmonic or sideband filtering or the like. In some embodiments, the demodulator **30** performs quadrature IQ demodulation. The intermediate frequency (IF) in some illustrative embodiments herein is about 120 Hz, although higher or lower IF is contemplated. The demodulated Doppler ultrasound signal **32** is digitized by a sample-and-hold analog-to-digital (A/D) converter **34** to generate a digitized demodulated Doppler ultrasound signal **40.** The A/D converter **34** may be embodied as an ADC IC and/or by discrete components, and the digitized demodulated Doppler ultrasound signal **40** output by the A/D converter **34** is read in by the at least one electronic processor **16** and may be stored in an internal memory (e.g. a solid state drive, SSD, or a flash memory, or so forth) and processed by the at least one electronic processor **16.** It is to be appreciated that the illustrative signal processing chain **30, 34** that generates the digitized demodulated Doppler ultrasound signal **40** is a simplified illustrative example. The signal processing chain may include variant and/or other operations or components such as signal conditioning, notch filtering to remove electrical line signal components and/or so forth, and moreover the processing may be variously partitioned between the analog or digital domains.

The digitized demodulated Doppler ultrasound signal **40** is processed by various DSP analyses **42** to generate clinically useful quantitative information. In the illustrative clinical task of fetal monitoring, the DSP analyses **42** may, for example, include fetal heartbeat detection and consequent derivation of the fetal heart rate, and fetal movement detection and consequent derivation of a fetal movement count per unit time (e.g. movements/minute). The DSP analyses **42** may also detect maternal heartbeats, fetal and/or maternal hiccups, and/or so forth in order to remove these confounding factors. The resulting clinical information (e.g. fetal heart rate, fetal movement count, a derived cardiac cycling trend line, and/or so forth) are suitably presented on the display 18 under control of the at least one electronic processor **16.**

With continuing reference to Fig. 1 and more particularly to the upper inset **26,** the at least one electronic processor **16** is further programmed to generate a higher pitched digitized demodulated Doppler ultrasound signal **50** from the digitized demodulated Doppler ultrasound signal **40** by signal segment downsampling operations **52** producing downsampled signal segments and duplication operations **54** that duplicate the downsampled signal segments. In the illustrative examples, the downsampling operations **52** downsample by a factor of *N* (where *N* is a positive integer greater than or equal to two), and the duplication operations **54** generate (*N* - 1) duplicates of each downsampled signal segment. For convenience of processing, the digitized demodulated Doppler ultrasound signal **40** is buffered in a buffering operation **56** into discrete buffers, e.g. each buffer of 0.5 seconds duration in some illustrative examples, and for each buffer a process of cycle detection **58** is performed to identify signal segments each consisting of a cycle of the intermediate frequency (IF) waveform of the digitized demodulated Doppler ultrasound signal which is then downsampled and duplicated as per the operations **52, 54.** Alternatively, it is contemplated to avoid buffering by introducing an acceptably short delay of, e.g. two IF cycles (16.7 msec) in an example, and processing the digitized demodulated Doppler ultrasound signal **40** using a sliding window of, e.g. 20 msec. Optionally, in some embodiments an initial DC component removal pre-processing operation **60** is performed; however, in some embodiments DC removal may be accomplished at an earlier point, e.g. by analog filtering performed by the demodulator **30.** The resulting higher pitched digitized Doppler ultrasound signal **50** is input to the audio chain **20, 22** for output by the loudspeaker **20.**

The DSP operations, including the DSP analyses **42** and the processing **52, 54, 56, 58, 60** to produce the higher pitched digitized Doppler ultrasound signal **50,** is suitably implemented by the at least one electronic processor **16** operatively connected with a non-transitory storage medium (not shown) that stores instructions which are readable and executable by the at least one electronic processor **16** to perform the disclosed DSP operations **42, 52, 54, 56, 58, 60.** The non-transitory storage medium may, for example, comprise a hard disk drive or other magnetic storage medium; a solid state drive, flash drive, electronically erasable programmable read-only memory (EEPROM) or other electronic memory storing firmware of the Doppler ultrasound device **10;** an optical disk or other optical storage; various combinations thereof; or so forth.

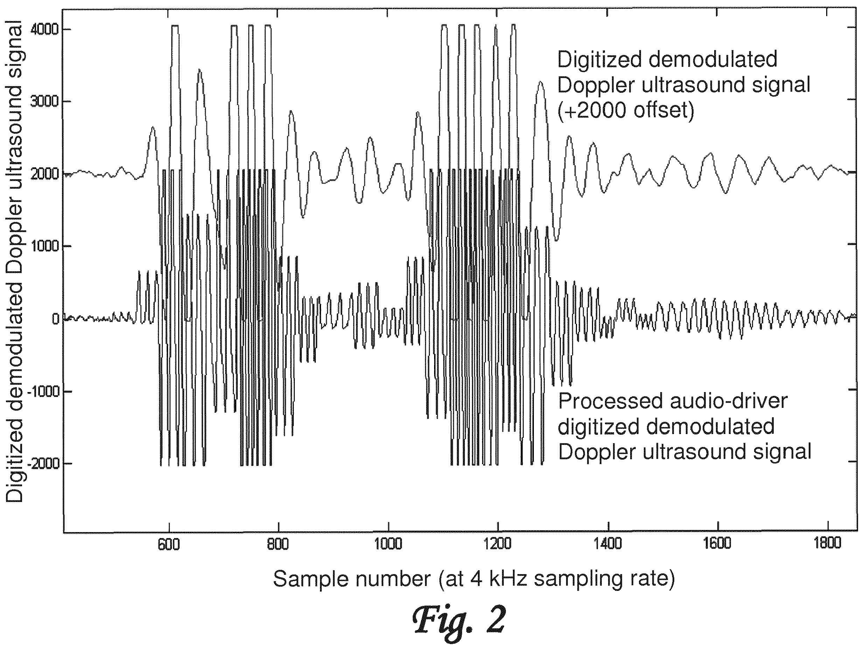

With reference now to Fig. 2, a plot is shown of a digitized demodulated Doppler ultrasound signal acquired by a Doppler ultrasound device for a fetal simulator (comprising a pump arranged inside a box to simulate a beating fetal heart, with the ultrasound probe placed on the surface to obtain the signals). Fig. 2 also plots a higher pitched digitized demodulated Doppler ultrasound signal obtained by detecting each cycle of the IF waveform (where in this example a cycle is detected as beginning at a negative-to-positive zero crossing and ending at the next negative-to-positive zero crossing), downsampling the detected cycle by a factor of $N = 3$ to generate a downsampled cycle, duplicating the downsampled cycle twice to fill a time interval of the detected cycle, and repeating the detecting, downsampling, and duplicating for each successive cycle of the IF waveform to generate the higher pitched digitized demodulated Doppler ultrasound signal. Note that in Fig. 2, to distinguish the digitized demodulated Doppler ultrasound signal from the higher pitched sample generated by the downsampling/duplicating, the digitized demodulated Doppler ultrasound signal is shown with an offset of +2000 in Fig. 2. It is readily seen that the effect of this processing is to boost the signal to a higher frequency band. In the example of Fig. 2, the digitized demodulated Doppler ultrasound signal is sampled at 4000 samples/sec, Fig. 2 shows a 0.5 sec interval (2000 samples, equivalent to 250 msec/sample), and the intermediate frequency (IF) is about 120 Hz which is at the low end of the audible frequency range (~20 Hz to 20 kHz).

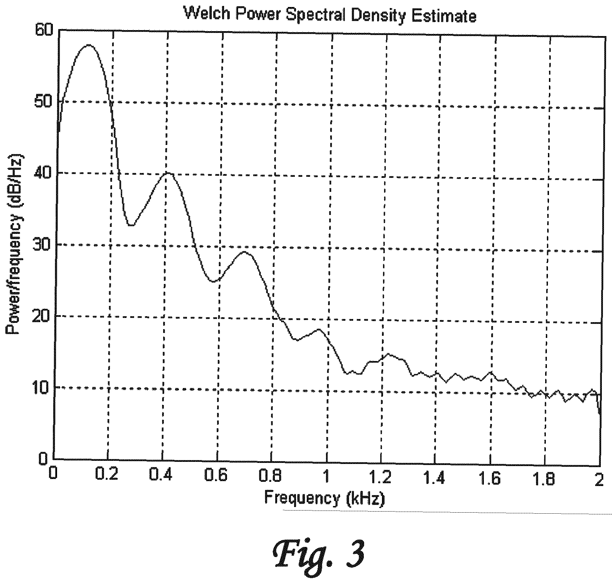

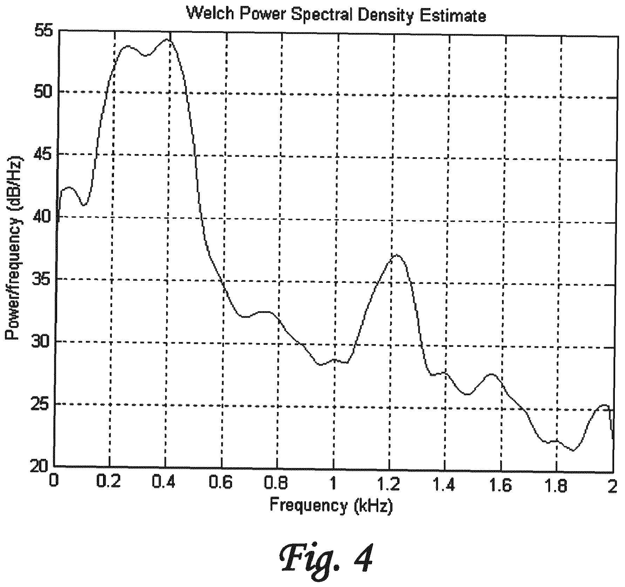

With reference to Figs. 3 and 4, Welch Power Spectral Density Estimates are shown for the original digitized demodulated Doppler ultrasound signal (Fig. 3) and for the higher pitched downsampled/duplicated waveform (Fig. 4). These spectra confirm that the original digitized demodulated Doppler ultrasound signal has a dominant frequency (the IF frequency) of about 120 Hz while the higher pitched digitized demodulated Doppler ultrasound signal has a higher dominant frequency corresponding to a higher audible pitch.

In the example illustrated with reference to Figs. 2-4, after the digitized demodulated Doppler ultrasound signal is acquired it is buffered in a buffer with a certain size, and the buffer is searched for complete cycles of the IF waveform in this buffer. In this example, the start point (sample) of the complete wave is defined as cross zero point which means that one sample is less than zero and the next sample is larger or equal to zero, or one sample is less than or equal to zero and next sample is larger than zero (i.e., a negative-to-positive zero crossing), and the end point of the waveform is the next negative-to-positive zero crossing. This detected cycle is downsampled by $N = 3$ to generate another the downsampled signal segment, which is duplicated twice (more generally, $N$ - 1 times). When using such a buffer approach, issues can arise if a cycle is not entirely contained within a single buffer. In a simple approach for handling this, if a complete cycle is not contained in a single buffer (because it crosses between two adjacent buffers), then the portion of the original digitized demodulated Doppler ultrasound signal **40** corresponding to the partial cycle is copied to the higher pitched digitized demodulated Doppler ultrasound signal **50.** The size of the buffer can be chosen to achieve a desired delay between signal acquisition and playback. For instance the delay is preferably around 20-50ms to make the playback (near) real-time. The sampling rate of the signal acquisition is another factor in choosing the buffer size. This processing will enhance the power at higher frequency range as seen by comparing Figs. 3 and 4, hence make the sound loud and clear and meanwhile keep the same fetal heart beat time invariant.

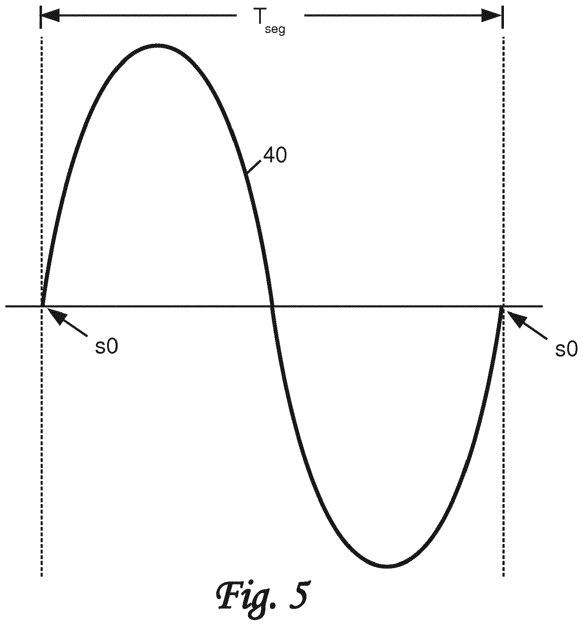

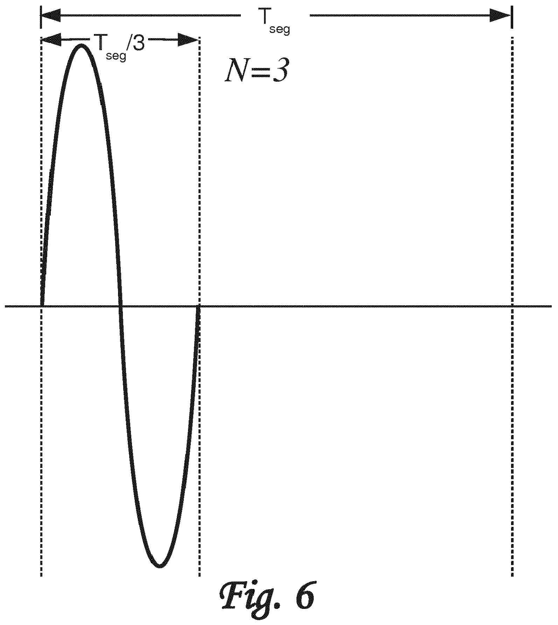

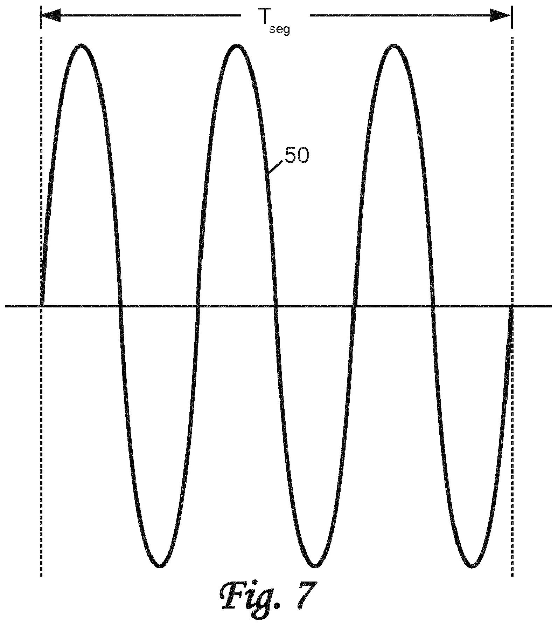

With reference to Figs. 5-7, an illustrative example of downsampling/duplication is described. Fig. 5 illustrates a detected cycle of the IF waveform, which extends from a start point **s0** which is a first negative-to-positive zero crossing to an end point **s1** which is an immediately next negative-to-positive zero crossing. The time interval of the detected cycle (or, more generally, a detected signal segment) is denoted as $T_{seg}$. Fig. 6 illustrates the result of the (1/$N$) downsampling **52** with $N = 3$. The amplitude of the cycle is unchanged, but its period is reduced to $T_{seg}/3$ (or, more generally, to $T_{seg}/N$). This leaves the remaining $2T_{seg}/3$ empty (or, more generally,

leaves the remaining $\dfrac{(N-1)T_{seg}}{N}$ empty). As shown in Fig. 7, this "empty" space is filled in by duplicating the downsampled cycle of Fig. 6 two times - or, more generally, duplicating the downsampled signal segment ($N$ - 1) times (for a total of $N$ copies of the downsampled cycle, including the original downsampled signal segment and the $N$ - 1 duplications). Comparing Figs. 5 and 7 readily demonstrates that the frequency has been tripled in this example. Advantageously, the amplitude is retained. As seen in Fig. 2, there is some "squaring off" of the amplitude in that the duplication results a peak being replaced by in $N$ peaks of the same height; however, since the informational content is generally at much lower frequency (e.g., the IF in this case is 120 Hz while the fetal heart rate is generally at about 2 Hz or lower, and fetal movements are likely to occur at sub-hertz rates; likewise, cardiac and blood flow cycling in echocardiography and vascular ultrasound procedures are usually in the low Hz to sub-Hz range).

The choice of detecting a cycle as extending from one negative-to-positive zero crossing **s0** to the next negative-to-positive zero crossing **s1** as in the example of Fig. 5 is advantageous since this results in smooth transitions at the interfaces between the downsampled signal segment and the duplicated signal segments as seen in Fig. 7. In other words, the final higher pitch signal of Fig. 7 is continuous and smooth at these interfaces. It will be appreciated that equivalent results are obtained if a cycle is detected as extending from a positive-to-negative zero crossing to the next positive-to-negative zero crossing. Furthermore, such zero crossings are readily detected: a negative-to-positive zero crossing can be detected by the condition s[n]>0 and s[n-1]≤0; and likewise a positive-to-negative zero crossing can be detected by the condition s[n]<0 and s[n-1]≥0.

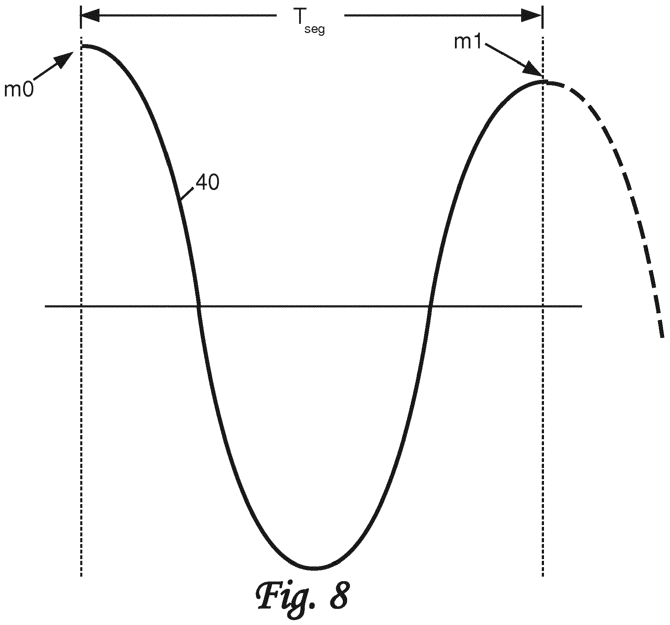

However, other signal segment definitions can be employed in detecting the signal segments, as shown in Figs. 8-14.

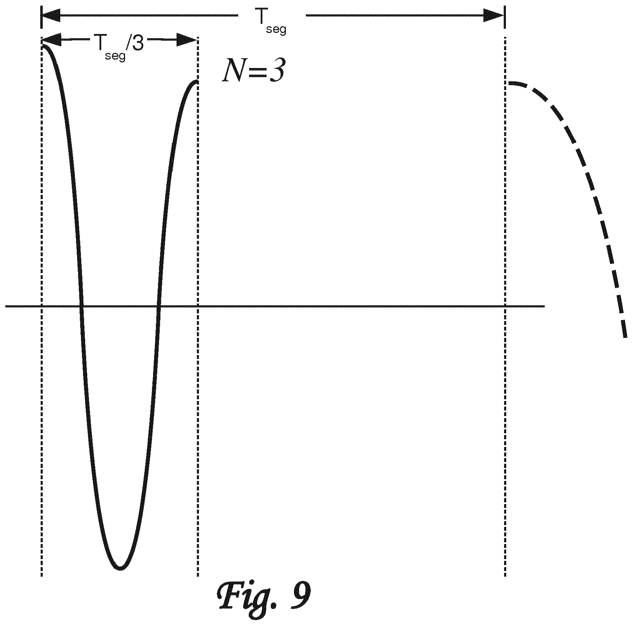

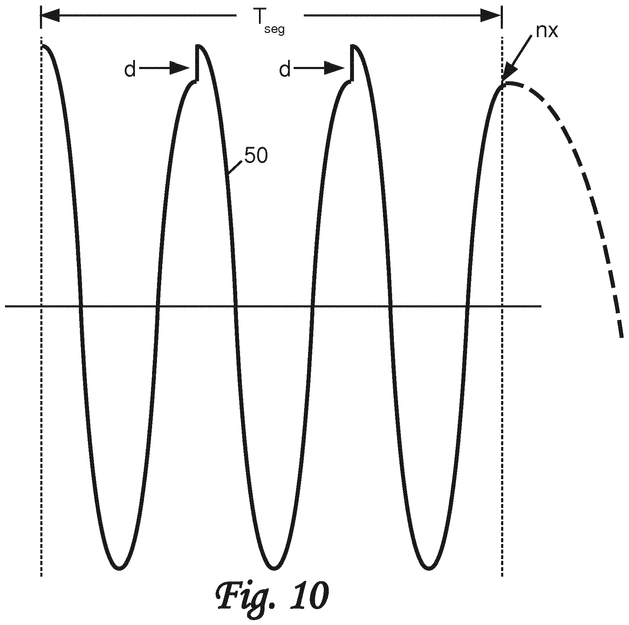

With reference to Figs. 8-10, in another example a signal segment is detected as extending from one maximum to the next maximum. Fig. 8 shows a cycle defined by the one maximum **m0** to a next maximum **m1.** Fig. 9 shows the downsampled signal segment, again using downsampling *of N* = 3. Fig. 10 shows the result after duplicating twice (more generally, duplicating (*N* - 1) times). As seen in Fig. 10, a disadvantage of using the maxima **m0, m1** as the detected cycle boundaries is that the beginning and ending maxima can be of different amplitude, leading to abrupt discontinuities **d** as indicated in Fig. 10. (Note, however, that the connection to the next part of the wave, denoted **nx** in Fig. 10, is smooth). These abrupt discontinuities **d** introduce spurious high frequency components into the higher pitched demodulated Doppler ultrasound signal **50** that is used for generating the audio signal (but not for the quantitative DSP analyses **42,** see Fig. 1), and hence these discontinuities **d** potentially can lead to high frequency "clicks" or other spurious sounds having an adverse effect on the perceived sound quality. One way to address this problem is to low pass or bandpass filter the output shown in Fig. 10 so as to remove these spurious high frequency components. In the approach of Figs. 8-10, a peak detector can be applied to find the maxima **m0, m1.** In a variant embodiment, it will be appreciated that a cycle can alternatively be similarly defined as extending from one minimum to a next minimum.

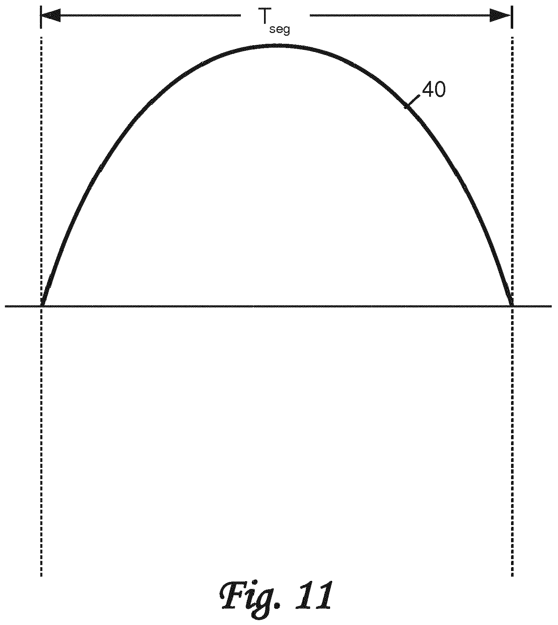

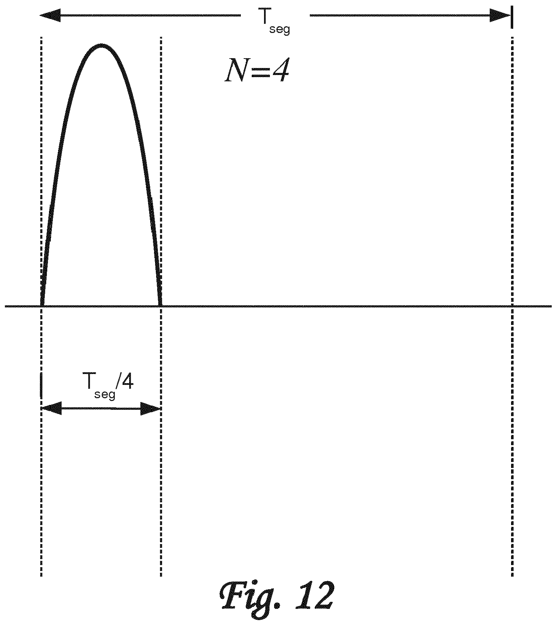

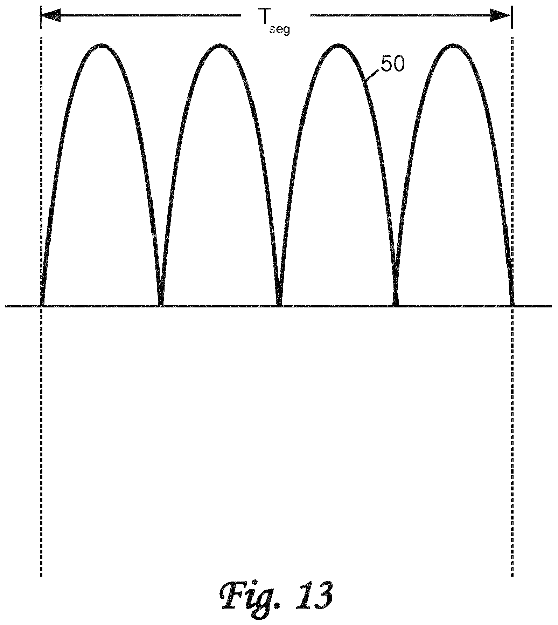

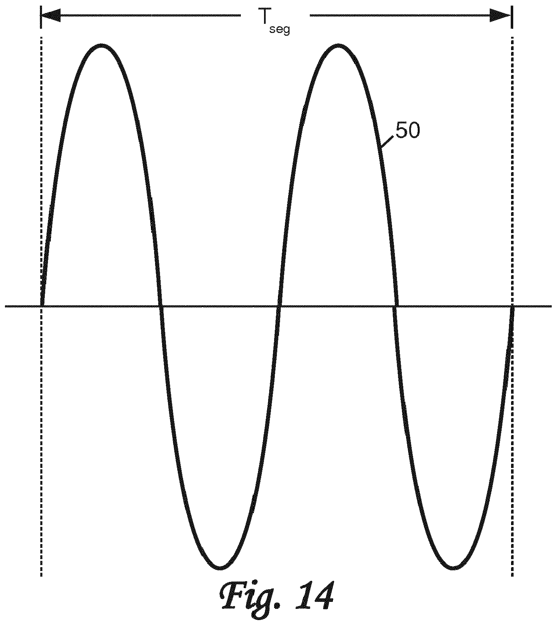

With reference to Figs. 11-14, in another embodiment the signal segments that undergo downsampling/duplication processing are cycle fractions, rather than whole cycles of the IF waveform. Fig. 11 illustrates a segment defined as extending from one zero crossing to the next zero crossing, without (in this embodiment) concern as to whether each zero crossing is negative-to-positive or positive-to-negative. As seen in Fig. 11, this results in the detected signal segment being a half-cycle. Fig. 12 shows the downsampling, in this case using *N* = 4. Thus, the downsampled segment only occupies one-fourth of the time interval $T_{seg}$ of the original detected half-cycle, and as seen in Fig. 13 this requires duplicating by (*N* - 1) = (4 - 1) = 3 times to fill the original time interval $T_{seg}$. While the resulting curve as seen in Fig. 13 is continuous, it is not differentiable (i.e., has abrupt change in slope) at the interfaces between the original downsampled half-cycle and the duplications. Since the resulting higher pitched demodulated Doppler ultrasound signal **50** is used for generating the audio signal but not for the quantitative DSP analyses **42** (see Fig. 1), these abrupt slope changes may not significantly degrade the audio quality. As illustrated in Fig. 14, in a variant embodiment the duplicating operation **54** optionally includes inverting the odd-numbered duplications (in this case, the first and third duplications) by multiplying by a factor of - 1. This results in smooth transitions as seen in Fig. 14.

With returning reference to Fig. 1, the choice of the downsampling factor *N* is suitably made to achieve the desired audio frequency. The downsampling factor *N* can be chosen based on the intermediate frequency (IF) of the demodulated signal **32** output by the demodulator **30** and the desired pitch of the higher pitched digitized demodulated Doppler ultrasound signal **50.** If the Doppler ultrasound device **10** employs a fixed intermediate frequency (IF), then the downsampling factor *N* can be hard-coded in the firmware of the Doppler ultrasound device **10.** On the other hand, if different ultrasound procedures employ different IF, then in a variant embodiment the electronic processor **16** is further programmed to implement an IF→*N* selector **66** that selects *N* based on the intermediate frequency (IF) currently set for the demodulator **30** and the desired pitch. This selector **66** can employ a simple look-up table, or an algorithm that (for example) chooses *N* given the input IF to set the output pitch as close as practicable to a target pitch (or more precisely the frequency corresponding to that target pitch) under a constraint that *N* is a positive integer. In similar fashion, it is contemplated to tailor the downsampling factor *N* to set the pitch to a desired value for a particular listener (e.g., a higher value of *N* may be set if the ultrasound operator has deficient hearing at lower frequencies) or for a particular procedure (e.g., different pitches may be preferred for fetal monitoring, echocardiography, or vascular ultrasound procedures). As yet another contemplated approach, a pitch control knob (not shown) can be provided on the Doppler ultrasound device **10,** by which a user can set the pitch - internally, adjusting the pitch control knob results in adjusting the value *of N.*

The invention has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

**Claims**

1. A Doppler ultrasound method for improving signal quality of a signal to be played, the method comprising:

   detecting (58) a cycle of a digitized demodulated Doppler ultrasound signal (40);
   downsampling (52) the detected cycle to generate a downsampled cycle;
   duplicating (54) the downsampled cycle to fill a time interval ($T_{seg}$) of the detected cycle; and
   repeating the detecting, downsampling, and duplicating for a plurality of cycles to generate a higher pitched digitized demodulated Doppler ultrasound signal (50).

2. The method of claim 1 wherein the downsampling (52) is by a factor *of N* where *N* is a positive integer with a value of $N = 2$ or higher, and the duplicating (54) comprises generating duplications of the downsampled cycle to fill the time interval ($T_{seg}$) of the detected cycle.

3. The method of any one of claims 1-2 wherein the detecting (58) comprises one of:

> detecting a cycle of the digitized demodulated Doppler ultrasound signal (40) extending from one negative-to-positive zero crossing to the next negative-to-positive zero crossing; or detecting a cycle of the digitized demodulated Doppler ultrasound signal (40) extending from one positive-to-negative zero crossing to the next positive-to-negative zero crossing.

4. The method of any one of claims 1-3 further comprising:

> outputting the higher pitched digitized demodulated Doppler ultrasound signal (50) to a loudspeaker (20) to generate an audible Doppler ultrasound signal.

5. The method of any one of claims 1-4 further comprising:

> performing digital signal processing (DSP) (42) on the digitized demodulated Doppler ultrasound signal (40) to determine quantitative fetal information including at least one of a fetal heart rate and a fetal movement count; and displaying the quantitative fetal information on a display (18).

6. The method of any one of claims 1-5 further comprising:

> buffering (56) the digitized demodulated Doppler ultrasound signal (40) into buffers each of a fixed time duration, wherein the detecting (58), downsampling (52), duplicating (54), and repeating are performed for each buffer; and for a partial cycle that is not contained in a single buffer, copying the portion of the digitized demodulated Doppler ultrasound signal (40) corresponding to the partial cycle to the higher pitched digitized demodulated Doppler ultrasound signal (50).

7. The method of any one of claims 1-6 further comprising:

> demodulating (30) and digitizing (34) a Doppler ultrasound signal (28) to generate the digitized demodulated Doppler ultrasound signal (40) at an intermediate frequency;

> setting the intermediate frequency of the demodulating (30); and selecting (66) the factor of $N$ for the downsampling (52) on the basis of the intermediate frequency to set a pitch of the higher pitched digitized demodulated Doppler ultrasound signal (50).

8. A Doppler ultrasound device (10) comprising:

> at least one electronic processor (16); and at least one non-transitory storage medium storing instructions readable and executable by the at least one electronic processor to perform an audio signal quality improvement method including:

>> detecting (58) a cycle of a digitized demodulated Doppler ultrasound signal (40); downsampling (52) the detected cycle to generate a downsampled cycle; duplicating (54) the downsampled cycle to fill a time interval ($T_{seg}$) of the detected cycle; and repeating the detecting, downsampling, and duplicating for a plurality of cycles to generate a higher pitched digitized demodulated Doppler ultrasound signal (50).

9. A medical device comprising:

> an ultrasound transducer (12) configured to acquire a Doppler ultrasound signal (28); a Doppler ultrasound device (10) operatively connected with the ultrasound transducer to generate a digitized demodulated Doppler ultrasound signal (40) from the acquired Doppler ultrasound signal and including at least one electronic processor (16) programmed to generate a higher pitched digitized demodulated Doppler ultrasound signal (50) from the digitized demodulated Doppler ultrasound signal by signal segment downsampling operations (52) producing downsampled signal segments and duplication operations (54) that duplicate the downsampled signal segments; and a loudspeaker (20) connected to output the higher pitched digitized demodulated Doppler ultrasound signal as an audible Doppler ultrasound signal.

10. The medical device of claim 9 wherein each downsampling operation (52) downsamples a signal segment occupying a time interval by a factor of $N$ where $N$ is a positive integer greater than or equal to two, and duplication operations (54) generate duplicates of the downsampled signal segment to fill the time interval of the signal segment.

**11.** The medical device of any one of claims 9-10 wherein each downsampling operation (52) includes:

detecting a signal segment consisting of a cycle of the digitized demodulated Doppler ultrasound signal (40); and

downsampling the detected cycle to produce a downsampled signal segment consisting of a downsampled cycle.

**12.** The medical device of claim 11 wherein the detecting comprises:

detecting each signal segment consisting of a cycle of the digitized demodulated Doppler ultrasound signal (40) by detecting successive negative-to-positive zero crossings or by detecting successive positive-to-negative zero crossings.

**13.** The medical device of any one of claims 9-12 further comprising:

a display (18);

wherein the at least one electronic processor (16) is further programmed to perform digital signal processing (DSP) (42) on the digitized demodulated Doppler ultrasound signal (40) to determine quantitative fetal information including at least one of a fetal heart rate and a fetal movement count and to operate the display to present the quantitative fetal information.

**14.** The medical device of any one of claims 9-13 wherein:

the Doppler ultrasound device (10) generates the digitized demodulated Doppler ultrasound signal (40) by operations including demodulating the acquired Doppler ultrasound signal (28) to an intermediate frequency, and

the downsampling operations (52) downsample by a factor of $N$ where $N$ is a positive integer greater than or equal to two, and

the at least one electronic processor (16) is further programmed to set the factor *of N* based on the intermediate frequency to set a pitch for the higher pitched digitized demodulated Doppler ultrasound signal (50).

**15.** The medical device of any one of claims 9-14 wherein the at least one electronic processor (16) is further programmed to:

buffer (56) the digitized demodulated Doppler ultrasound signal (40) into buffers each of a fixed time duration, wherein the signal segment downsampling operations (52) produce downsampled signal segments comprising cycles of the digitized demodulated Doppler ultrasound signal and the downsampling and duplication operations (52, 54) are performed for each buffer;

wherein the higher pitched digitized demodulated Doppler ultrasound signal (50) is generated from the digitized demodulated Doppler ultrasound signal (40) by the downsampling and duplication operations and by a further operation of copying a portion of the digitized demodulated Doppler ultrasound signal corresponding to a partial cycle that is not contained in a single buffer to the higher pitched digitized demodulated Doppler ultrasound signal.

Fig. 1

*Fig. 2*

Welch Power Spectral Density Estimate

*Fig. 3*

Welch Power Spectral Density Estimate

*Fig. 4*

EP 3 505 072 A1

Fig. 5

$$T_{seg}$$

$$T_{seg}/3$$

$$N=3$$

*Fig. 6*

EP 3 505 072 A1

*Fig. 7*

Fig. 8

Fig. 9

*Fig. 10*

EP 3 505 072 A1

Fig. 11

Fig. 12

Fig. 13

EP 3 505 072 A1

Fig. 14

**Europäisches Patentamt**
**European Patent Office**
**Office européen des brevets**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 595 921 B1 (URBANO JOSEPH A [US] ET AL) 22 July 2003 (2003-07-22) * column 1, lines 14-26; claims; figures * * column 3, line 30 - column 5, line 54 * * column 32, line 5 - column 34, line 39 * | 1-15 | INV. A61B8/08 |
| A | WO 2007/023438 A2 (KONINKL PHILIPS ELECTRONICS NV [NL]; CLARK DAVID W [US]) 1 March 2007 (2007-03-01) * the whole document * | 1-15 | |
| A | WO 2010/131136 A1 (KONINKL PHILIPS ELECTRONICS NV [NL]; AGARWAL ANUP [US]; HOPE SIMPSON D) 18 November 2010 (2010-11-18) * the whole document * | 1-15 | |
| A | CN 104 887 269 A (SUNRAY MEDICAL APPARATUS CO LTD) 9 September 2015 (2015-09-09) * the whole document * | 1-15 | |
| A | WO 2010/035022 A1 (HUNTLEIGH TECHNOLOGY LTD [GB]; JACKSON ROY [GB]) 1 April 2010 (2010-04-01) * the whole document * | 1-15 | TECHNICAL FIELDS SEARCHED (IPC) A61B |

The present search report has been drawn up for all claims

| Place of search | Date of completion of the search | Examiner |
|---|---|---|
| Munich | 15 June 2018 | Mundakapadam, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 21 0910

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2018

| Patent document cited in search report | | Publication date | Patent family member(s) | | Publication date |
|---|---|---|---|---|---|
| US 6595921 | B1 | 22-07-2003 | NONE | | |
| WO 2007023438 | A2 | 01-03-2007 | EP | 1922563 A2 | 21-05-2008 |
| | | | US | 2009131791 A1 | 21-05-2009 |
| | | | WO | 2007023438 A2 | 01-03-2007 |
| WO 2010131136 | A1 | 18-11-2010 | CN | 102421372 A | 18-04-2012 |
| | | | EP | 2429405 A1 | 21-03-2012 |
| | | | JP | 5642159 B2 | 17-12-2014 |
| | | | JP | 2012526584 A | 01-11-2012 |
| | | | RU | 2011150512 A | 20-06-2013 |
| | | | US | 2012059264 A1 | 08-03-2012 |
| | | | US | 2017307742 A1 | 26-10-2017 |
| | | | WO | 2010131136 A1 | 18-11-2010 |
| CN 104887269 | A | 09-09-2015 | NONE | | |
| WO 2010035022 | A1 | 01-04-2010 | AU | 2009295644 A1 | 01-04-2010 |
| | | | CA | 2737876 A1 | 01-04-2010 |
| | | | CN | 102164544 A | 24-08-2011 |
| | | | DK | 2346408 T3 | 21-07-2014 |
| | | | EP | 2346408 A1 | 27-07-2011 |
| | | | JP | 5634999 B2 | 03-12-2014 |
| | | | JP | 2012502717 A | 02-02-2012 |
| | | | US | 2011172540 A1 | 14-07-2011 |
| | | | WO | 2010035022 A1 | 01-04-2010 |

EPO FORM P0459

For more details about this annex : see Official Journal of the European Patent Office, No. 12/82